# EUROPEAN PATENT SPECIFICATION

(11) **EP 2 307 031 B1**
(45) Date of publication and mention of the grant of the patent: **29.07.2020**
(21) Application number: 09804542.0
(22) Date of filing: 29.07.2009
(51) Int. Cl.: A61K 31/722, A61K 47/36, A61K 31/506, A61K 31/56, A61K 31/585, A61K 8/34, A61K 8/49, A61Q 5/00, A61K 8/63, A61K 8/73, A61K 8/97, A61K 9/00, A61Q 7/00

(54) **FINASTERIDE FORMULATIONS FOR DRUG RELEASE TO HAIR AND SCALP**
FINASTERID-FORMULIERUNGEN ZUR WIRKSTOFFFREISETZUNG AN HAAR UND KOPFHAUT
FORMULATIONS DE FINASTÉRIDE POUR LA LIBÉRATION DE MÉDICAMENTS DANS LES CHEVEUX ET LE CUIR CHEVELU

(30) Priority: 04.08.2008 EP 08161757
(43) Date of publication of application: 13.04.2011
(60) Divisional application: 20180024.0
(73) Proprietor: Polichem SA, L-1526 Luxembourg (LU)
(72) Inventor: MAILLAND, Federico, CH-6900 Lugano (CH); MURA, Emanuela, I-22100 Como (IT)
(74) Representative: J A Kemp LLP
(86) International application number: PCT/EP2009/059807
(87) International publication number: WO 2010/015556

(56) References cited:
- EP-A- 1 491 202
- WO-A-02/07683
- US-A1- 2002 192 280
- BIRUSS ET AL: "Skin permeation of different steroid hormones from polymeric coated liposomal formulations" EUROPEAN JOURNAL OF PHARMACEUTICS AND BIOPHARMACEUTICS, ELSEVIER SCIENCE PUBLISHERS B.V., AMSTERDAM, NL, vol. 62, no. 2, 1 February 2006 (2006-02-01), pages 210-219, XP025138691 ISSN: 0939-6411

## Description

The present invention relates to liquid compositions containing a chitosan derivative or a physiologically acceptable salt thereof, for the preparation of a medicament, or a medical device, or a sanitary product, or a cosmetic, forming a film after application onto the scalp, or onto the hair, useful for delivery of actives onto the scalp surface and on the hair. Delivery of active ingredients to scalp or the hair, contained in drugs, sanitary products, detergents or cosmetics, often constitutes a problem in that the common formulations, like cream, ointment, gel, powder, or foam, do not allow a long lasting contact with the scalp or hair surface.

Chitosan and its derivatives are amino-polysaccharides, derived from the chitin extracted from the exoskeleton of the crustaceans, known in the art for their use in different preparations. KR20020084672 discloses chitosan as an ingredient of microspheres, useful as a carrier for separation of proteins or peptides; KR20020048534 reports chitosan as an ingredient of a pack composition for skin massage, including paraffin wax as an effective component; JP2005306746 is teaching the use of chitosan to obtain a wrinkle therapeutic agent as an ingredient of gel-like or spongy preparations of botulinus toxin. WO2005055924 reports chitosan derivatives as ingredients of hydrogels useful for cavity-filling wound dressings. JP2004231604 teaches compositions of chitosans having a high deacetylation degree, as an ingredient of a carrier sheet with a porous spongy texture. WO03042251 discloses compositions comprising chitosan in the form of a network of nano-sized fibres. WO02057983 discloses a multilayered, air gap sheet of chitosan with a regular lamellar structure which retains drugs for a prolonged period of time; JP11060605 teaches an amphiphilic chitosan derivative which can be used as dispersion stabilizer or emulsifier in a drug for application to skin. Finally, EP1303249, discloses a nail varnish composition containing at least one antimycotic agent and an hydroxyalkyl or a carboxyalkyl chitosan, whereas WO2004/112814 discloses a nail restructuring composition based on one herb extract from the genus Equisetum in combination with hydroxypropyl chitosan.

It has now surprisingly been found that liquid preparations of a chitosan derivative which is hydroxypropyl-chitosan, besides being useful for the application to nails, may form an elastic film, after application to the scalp and after evaporation of the volatile solvents, suitable to maintain drugs and other actives in intimate contact to the scalp and/or hair surface. The film forming solutions containing hydroxypropyl-chitosan, pharmaceutically active agents and volatile solvents, may easily be sprayed onto the scalp or hair surface, by allowing quick evaporation of the volatile solvents and easy formation of an elastic film, that avoids, as an example, bothersome sensation of oily scalp compared to creams, lotions and ointments, and avoids long time waiting for drying, when large scalp surface is to be treated. The film forming solutions of hydroxypropyl-chitosan may also be applied on the scalp by gently massage, or by spray. The film forming solutions of hydroxypropyl-chitosan allow quick penetration of actives into the deep layers of the scalp, thus resulting useful for the safe treatment and/or prevention of scalp conditions and/or diseases, like hair loss, where penetration of the active ingredient until the hair follicles is needed. Moreover, the dermal film formed after evaporation of solvents of liquid hydroxypropyl-chitosan compositions allows long lasting intimate contact with the scalp and/or hair surface, and continuous release of drugs or other actives for many hours after the application.

### DESCRIPTION OF THE INVENTION

The invention provides a composition containing: (a) hydroxypropyl-chitosan or a physiologically acceptable salt thereof in an amount of from 0.25 to 2.0% with respect to the total weight of the composition; (b) finasteride; (c) at least one volatile solvent selected from ethanol or isopropanol in an amount of from 35% to 80% with respect to the total weight of the composition; and (d) water, for use in the treatment and/or prevention of scalp and/or hair conditions and/or diseases.

The film forming solutions of hydroxypropyl-chitosan are topically applied to the scalp and/or hair of the recipient, preferably a human being; they may be applied by a gently massage on the scalp or may easily be sprayed by allowing the formation of an elastic film. The film forming solutions of hydroxypropyl-chitosan allow quick penetration of finasteride, a long lasting intimate contact with the scalp and continuous release of drugs for many hours after the application.

Liquid preparations object of this invention are typically in the form of solutions, emulsions, colloids or suspensions, with finasteride from 0.001 to 25 wt.%, more preferably from 0.2 to 10 wt.%, most preferably from 0.4 to 5.0 wt.%, suitable to form an elastic film after evaporation of solvent, said film being in intimate contact with the scalp surface and/or of the hair and allowing quick and long lasting penetration of said actives useful for treating scalp and/or hair conditions and/or diseases, like hair loss, baldness, alopecia, androgenetic alopecia, hair fragility and other hair conditions.

Compositions object of this invention are superior to the conventional formulations, in that they can be sprayed onto the surface, leaving an uniform and invisible film. Moreover, compositions according to the present invention do not dirty, do not dry like gels and lotions do, and do not give bothersome sensation when applied, like other rigid film preparations do.

Pharmaceutical compositions are prepared according to conventional technique, using compatible excipients and pharmaceutically acceptable carriers, and may contain, in combination, other active principles with complementary or, in any case, useful activity.

Examples of these compositions prepared according to the present invention include: solutions, emulsions, suspensions, colloids, for application to nude or hairy scalp.

The compositions according to the present invention may further contain one or more active agents selected from 5-alpha reductase inhibitors, antiandrogenic hormones, potassium chanel agonists, aminoacids, plant extracts, antioxidants, and are suitable to prevent and to treat hair loss, baldness, alopecia; to nourish, volumize and reinforce the hair.

Examples of 5-alpha reductase inhibitors which may be included in the composition in accordance with the present invention include: dutasteride, azelaic acid, betasitosterol, zinc, Vitamin B6.

Examples of antiandrogenic hormones which may be included in the composition in accordance with the present invention include: spironolactone, ciproterone acetate, flutamide, ketoconazole, oestrogens and their salts.

Examples of potassium chanel agonists which may be included in the composition in accordance with the present invention include minoxidil.

Examples of aminoacids which may be included in the composition in accordance with the present invention include: L-cysteine, N-acetylcisteine, L-cystine, L- methionine, dimethylsulphone, L-taurine.

Examples of plants which extracts may be included in the composition in accordance with the present invention include: Serenoa repens , Aloe vera, Equisetum arvense, Panicum miliaceum, Pygeum africanum, Urtica dioica, Coix lachrymal-j obi, Eriobotrya Japonica.

Examples of antioxidants which may be included in the composition in accordance with the present invention include: ascorbic acid; glutathione; melatonin; tocopherols and tocotrienols ; polyphenolic antioxidants including resveratrol and flavonoids; viniferols; carotenoids including lycopene, carotenes.

The compositions according to the invention may be applied onto the scalp surface by gently massage of the concerned area, or onto the hair by spray. After evaporation, an elastic film is formed onto the treated surface, that allows the continuous delivery of the actives to the scalp and/or the hair for many hours or even for days. Compositions object of this invention may also contain penetration modifying systems, including absorption enhancers, or modified delivery systems, which modify the penetration rate of the actives into the epidermis and create a reservoir at the dermal-epidermal junction, to provide an increased and longer lasting availability of the active at the hair follicles. Examples of the absorption enhancers which may be included in the composition in accordance with the present invention include Transcutol P® (diethyleneglycol monoethylether). Examples of modified delivery systems which may be included in the composition in accordance with the present invention include microcapsules.

The pharmaceutical compositions and the uses of the present invention will now be more fully described by the following examples.

### EXAMPLE 1

A film forming solution having the following composition wt./wt.% was prepared:

| | | |
|---|---|---|
| 1) | Finasteride | 0.25% |
| 2) | Ethyl Alcohol 96° | 55.00% |
| 3) | Propylene Glycol | 5.00% |
| 4) | Hydroxypropyl Chitosan | 1.00% |
| 5) | Purified water | 38.75% |

### Preparation

Ethyl Alcohol, Propylene Glycol and water were mixed at room temperature. Finasteride was then added and mixed until a clear solution was obtained. Hydroxypropyl Chitosan was added as the final ingredient, and the mixture was stirred at room temperature for 24 hours or until dissolution.

The obtained formulation was a clear and colourless solution, homogenous in appearance even after prolonged storage. Moreover, the liquid was able to form a matte, non-sticky and elastic film, which could strongly adhere to the scalp surface.

### EXAMPLE 2 (REFERENCE EXAMPLE)

A liquid formulation having the following w/w % composition was prepared:

| | | |
|---|---|---|
| 1) | Dutasteride | 0.25% |
| 2) | Ethyl Alcohol 96° | 55.00% |
| 3) | Propylene Glycol | 5.00% |
| 4) | Hydroxypropyl Chitosan | 2.00% |
| 5) | Purified water | 37.75% |

### Preparation

The formulation was prepared by using the same method described for Example 1.

Hydroxypropyl Chitosan was added as the final ingredient, and the mixture was stirred at room temperature for 24 hours or until dissolution.

The obtained formulation was a clear and colourless solution, homogenous in appearance even after prolonged storage. Moreover, the liquid was able to form a matte, non-sticky and elastic film, which could strongly adhere to the scalp surface.

### EXAMPLE 3 (REFERENCE EXAMPLE)

A liquid formulation having the following w/w % composition was prepared:

| | | |
|---|---|---|
| 1) | Minoxidil | 2.00% |
| 2) | Ethyl Alcohol 96° | 55.00% |
| 3) | Propylene Glycol | 5.00% |
| 4) | Hydroxypropyl Chitosan | 1.00% |
| 5) | Purified water | 37.00% |

### Preparation

The formulation was prepared by using the same method described for Example 1.

Hydroxypropyl Chitosan was added as the final ingredient, and the mixture was stirred at room temperature for 24 hours or until dissolution. The obtained formulation was a clear and colourless solution, homogenous in appearance even after prolonged storage. Moreover, the liquid was able to form a matte, non-sticky and elastic film, which could strongly adhere to the scalp surface.

### EXAMPLE 4 (REFERENCE EXAMPLE)

A liquid formulation having the following w/w % composition was prepared:

| | | |
|---|---|---|
| 1) | Spironolactone | 1.00% |
| 2) | Ethyl Alcohol 96° | 55.00% |
| 3) | Propylene Glycol | 5.00% |
| 4) | Hydroxypropyl Chitosan | 2.00% |
| 5) | Purified water | 37.00% |

### Preparation

The formulation was prepared by using the same method described for Example 1.

Hydroxypropyl Chitosan was added as the final ingredient, and the mixture was stirred at room temperature for 24 hours or until dissolution.

The obtained formulation was a clear and colourless solution, homogenous in appearance even after prolonged storage. Moreover, the liquid was able to form a matte, non-sticky and elastic film, which could strongly adhere to the scalp surface.

### EXAMPLE 5

An in vitro permeation test was performed by applying the film forming solution according to the Example 1 to excised hairless rat skin, obtained from dorsal or abdominal skin of male hairless rats. Portions of the skin (ca 9 cm²), after removal of adhering fat and subcutaneous tissues, were placed as a barrier between the two compartment of Gummer permeation vertical cells (Gummer, CL. et al. The skin penetration cell: design update. Int. J. Pharm. 1987, 40, 101-104). The receiving phase was introduced into the lower compartment and 1.0 or 0.5 mL of the composition according to the Example 1 were regularly distributed on the exposed skin surface. At predetermined time intervals (2, 4, 8, 12, 16, 20 and 24 hours) 5.0 mL of the receiving solution were collected for analysis and immediately replaced by an equal volume of fresh buffer. The experiment was replicated 3 times.

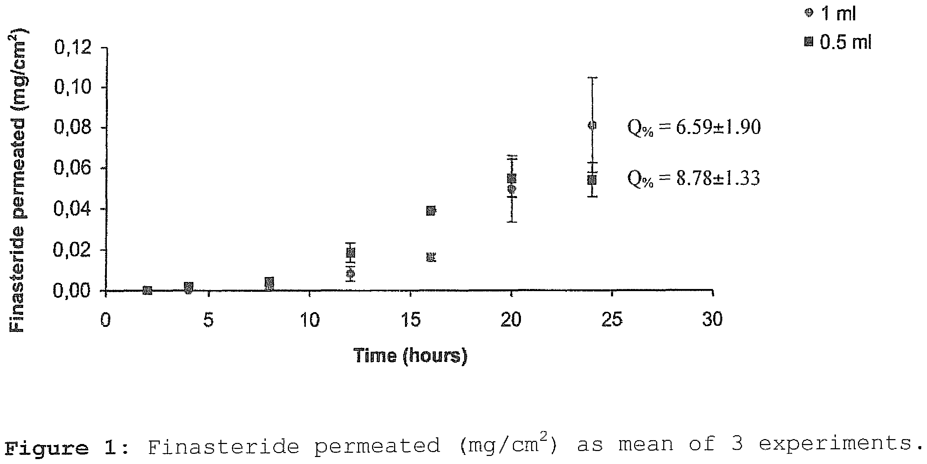

The finasteride permeated through hairless rat skin in the 3 experiments is reported in Figure 1. The total percent quantity (Q%) permeated through the rat hairless skin was 6.59 ± 1.90% for the 1.0 mL dose and 8.78 ± 1.33% for the 0.5 mL dose.

It is concluded that finasteride was able to permeate the rat skin, after the application of the film forming solution of hydroxypropyl chitosan according to the Example 1, in a quick and long lasting way.

### EXAMPLE 6 (REFERENCE EXAMPLE)

A preparation having the following composition wt./wt.% was prepared:

| | | |
|---|---|---|
| 1. | finasteride | 0.25% |
| 2. | purified water | 19.25% |
| 3. | propylene glycol | 10.00% |
| 4. | isopropanol | 70.00% |
| 5. | chitosan | 0.50% |

### Preparation

The formulation was prepared by dissolving chitosan and finasteride in propylene glycol, then adding the other ingredients, and stirring the mixture until dissolution. The resulting liquid was able to form an elastic film which could strongly adhere to the skin surface.

### EXAMPLE 7 (REFERENCE EXAMPLE)

A liquid formulation having the following w/w % composition was prepared:

| | | |
|---|---|---|
| 1. | *Eriobotrya Japonica* Leaf *1,3-butylene glycol extract*¹ | 10.00% |
| 2. | Ethyl Alcohol 96° | 36.50% |
| 3. | Diethyleneglycol monoethylether² | 0.50% |
| 4. | Hydroxypropyl Chitosan | 1.00% |
| 5. | Purified water | 52.00% |

| | | |
|---|---|---|
| ¹Loquat Leaf Extract CA; ²Transcutol® | | |

### Preparation

Ethyl Alcohol and water were mixed at room temperature. The Loquat Leaf Extract CA was then added and mixed until a clear solution was obtained. Diethyleneglycol monoethylether was added; Hydroxypropyl Chitosan was added as the final ingredient, and the mixture was stirred at room temperature for 4 hours or until dissolution. The obtained formulation was a clear and colourless solution, homogenous in appearance. Moreover, the liquid was able to form a matte, non-sticky and elastic film.

### EXAMPLE 8 (REFERENCE EXAMPLE)

A liquid formulation having the following w/w % composition was prepared:

| | | |
|---|---|---|
| 1*.* | *Coix lachrymal-jobi 1,3-butylene glycol solution*¹ | 10.00% |
| 2. | Ethyl Alcohol 96° | 36.50% |
| 3. | Diethyleneglycol monoethylether² | 0.50% |
| 4. | Hydroxypropyl Chitosan | 1.00% |
| 5. | Purified water | 52.00% |

| | | |
|---|---|---|
| ¹Hydrolyzed Coix Extract; ²Transcutol® P | | |

### Preparation (REFERENCE EXAMPLE)

Ethyl Alcohol and water were mixed at room temperature. The Hydrolyzed Coix Extract was then added and mixed until a clear solution was obtained. Diethyleneglycol monoethylether was added; Hydroxypropyl Chitosan was added as the final ingredient, and the mixture was stirred at room temperature for 4 hours or until dissolution.

The obtained formulation was a clear and slightly yellow solution, homogenous in appearance. Moreover, the liquid was able to form a matte, non-sticky and elastic film.

### EXAMPLE 9 (REFERENCE EXAMPLE)

A liquid formulation having the following w/w % composition was prepared:

| | | |
|---|---|---|
| *1)* | *Eriobotrya Japonica Leaf 1,3-butylene glycol extract*¹ | 10.00% |
| 2) | Ethyl Alcohol 96° | 36.50% |
| 3) | Diethyleneglycol monoethylether² | 0.50% |
| 4) | Chitosan | 1.00% |
| 5) | Purified water | 52.00% |

| | | |
|---|---|---|
| ¹Loquat Leaf Extract CA; ²Transcutol® P | | |

### Preparation

Chitosan was dissolved in water after acidification with acetic acid at pH 3.0. Then, ethyl alcohol was added and the mix was stirred till to obtain a clear viscous solution. At this point, the pH rose till to 5.5.

Diethyleneglycol monoethylether was added and, at the end the herbal extract.

The obtained formulation was a clear and colourless solution, homogenous in appearance and slightly viscous.

### EXAMPLE 10 (REFERENCE EXAMPLE)

An open, comparative test was performed to assess the effect of the solution according to the Example 9 on the hair of 4 healthy female volunteers, aged 20-45 years, who gave their informed consent. All the women had smooth hair, subject 1 natural, subjects 2 and 3 dyed, subject 4 with streaks. After washing the hair with a standard shampoo, two wisps were sampled from each woman, from the same area of the scalp, and the following procedures were followed:
One wisp of each woman was applied the solution according to the Example 9 and then blow-dried. The other wisp was blow-dried and served as untreated control.

The following parameters were measured:
1. Volume: 0=scarce, 1=poor, 2=moderate, 3=high volume
2. Resistance to traction (as per the UNI EN ISO2062:1997)
3. Spectrophotometry (shining was measured according to the CIE, Commission Internationale de l'Eclairage)
4. Digital 3D videomicroscopy by 3D Hirox KH-770at 2100x and 350x

The results are as follows: the treated wisps resulted with a higher mean volume by 12%, a higher shining measured by spectrophotometry by 6% and resistance to traction 11% higher than the control wisps. At the digital videomicroscopy the treated hairs appeared smoother with a more regular aspect of the cuticle.

In conclusion, the preparation as per the Example 9 was able to volumise the human hair, to reinforce them and to improve their shining and smoothness.

## Claims

1. A composition containing:
(a) hydroxypropyl-chitosan or a physiologically acceptable salt thereof in an amount of from 0.25 to 2.0% with respect to the total weight of the composition;
(b) finasteride;
(c) at least one volatile solvent selected from ethanol or isopropanol in an amount of from 35% to 80% with respect to the total weight of the composition; and
(d) water,
for use in the treatment and/or prevention of scalp and/or hair conditions and/or diseases.

2. A composition for use according to claim 1, wherein the composition comprises customary excipients and/or adjuvants.

3. A composition for use according to claim 1 or claim 2, wherein the composition is a liquid.

4. A composition for use according to claim 3, wherein the composition is a solution, emulsion, suspension or colloid.

5. A composition for use according to any one of claims 1 to 4, wherein the composition is applied as a spray.

6. A composition for use according to any one of the preceding claims, wherein said scalp and/or hair conditions and/or diseases are selected from hair loss, baldness, alopecia, androgenetic alopecia and hair fragility.

7. A composition for use according to any one of claims 1 to 6, which composition further contains at least a penetration modifying system.

8. A composition for use according to claim 7, wherein the at least a penetration modifying system is selected from diethyleneglycol monoethylether and microcapsules.

9. A composition for use according to any one of the preceding claims, wherein said treatment and/or prevention of scalp and/or hair conditions and/or diseases is achieved by applying said composition to the scalp and/or hair of a human being.

## Patentansprüche

1. Zusammensetzung, die Folgendes enthält:
(a) Hydroxypropylchitosan oder ein physiologisch annehmbares Salz davon in einer Menge von 0,25 bis 2,0 % in Bezug auf das Gesamtgewicht der Zusammensetzung;
(b) Finasterid;
(c) mindestens ein flüchtiges Lösungsmittel, ausgewählt aus Ethanol oder Isopropanol in einer Menge von 35 % bis 80 % in Bezug auf das Gesamtgewicht der Zusammensetzung; und
(d) Wasser,
zur Verwendung bei der Behandlung und/oder Prävention von Leiden und/oder Erkrankungen der Kopfhaut und/oder der Haare.

2. Zusammensetzung zur Verwendung nach Anspruch 1, wobei die Zusammensetzung herkömmliche Trägerstoffe und/oder Adjuvantien umfasst.

3. Zusammensetzung zur Verwendung nach Anspruch 1 oder Anspruch 2, wobei die Zusammensetzung eine Flüssigkeit ist.

4. Zusammensetzung zur Verwendung nach Anspruch 3, wobei die Zusammensetzung eine Lösung, Emulsion, Suspension oder ein Kolloid ist.

5. Zusammensetzung zur Verwendung nach einem der Ansprüche 1 bis 4, wobei die Zusammensetzung als ein Spray aufgetragen wird.

6. Zusammensetzung zur Verwendung nach einem der vorhergehenden Ansprüche, wobei die Leiden und/oder Erkrankungen der Kopfhaut und/oder der Haare ausgewählt sind aus Haarausfall, Kahlheit, Alopezie, androgenetischer Alopezie und Haarbrüchigkeit.

7. Zusammensetzung zur Verwendung nach einem der Ansprüche 1 bis 6, wobei die Zusammensetzung ferner mindestens ein Penetrationsmodifikationssystem enthält.

8. Zusammensetzung zur Verwendung nach Anspruch 7, wobei das mindestens eine Penetrationsmodifikationssystem ausgewählt ist aus Diethylenglykolmonoethylether und Mikrokapseln.

9. Zusammensetzung zur Verwendung nach einem der vorhergehenden Ansprüche, wobei die Behandlung und/oder Prävention von Leiden und/oder Erkrankungen der Kopfhaut und/oder der Haare durch Auftragen der Zusammensetzung auf die Kopfhaut und/oder die Haare eines Menschen erreicht wird.

## Revendications

1. Composition contenant :
(a) de l'hydroxypropyl-chitosane ou un sel physiologiquement acceptable de celui-ci en une quantité de 0,25 à 2,0% par rapport au poids total de la composition ;
(b) de la finastéride ;
(c) au moins un solvant volatil choisi parmi l'éthanol ou l'isopropanol en une quantité de 35% à 80% par rapport au poids total de la composition; et
(d) de l'eau,
destinée à être utilisée dans le traitement et / ou la prévention des affections et / ou des maladies du cuir chevelu et / ou des cheveux.

2. Composition à utiliser selon la revendication 1, dans laquelle la composition comprend des excipients et / ou des adjuvants habituels.

3. Composition à utiliser selon la revendication 1 ou la revendication 2, dans laquelle la composition est un liquide.

4. Composition à utiliser selon la revendication 3, dans laquelle la composition est une solution, une émulsion, une suspension ou un colloïde.

5. Composition à utiliser selon l'une quelconque des revendications 1 à 4, dans laquelle la composition est appliquée sous forme de spray.

6. Composition à utiliser selon l'une quelconque des revendications précédentes, dans laquelle lesdites conditions et / ou maladies du cuir chevelu et / ou des cheveux sont choisies parmi la perte de cheveux, la calvitie, l'alopécie, l'alopécie androgénétique et la fragilité des cheveux.

7. Composition à utiliser selon l'une quelconque des revendications 1 à 6, laquelle composition contient en outre au moins un système modifiant la pénétration.

8. Composition à utiliser selon la revendication 7, dans laquelle l'au moins un système modifiant la pénétration est choisi parmi le monoéthyléther de diéthylèneglycol et les microcapsules.

9. Composition à utiliser selon l'une quelconque des revendications précédentes, dans laquelle ledit traitement et / ou prévention des affections et / ou des maladies du cuir chevelu et / ou des cheveux est obtenu en appliquant ladite composition au cuir chevelu et / ou aux cheveux d'un être humain.
